# EUROPEAN PATENT APPLICATION

(11) **EP 1 604 997 A1**
(43) Date of publication of application: **14.12.2005**
(21) Application number: 05018086.8
(22) Date of filing: 04.02.2000
(51) Int. Cl.: C07K 16/08, C07K 16/18, C07K 16/28, C07K 16/30, C07K 16/24, A61K 39/395, A01K 67/027

(54) **Human polyclonal antibodies from transgenic nonhuman animals**

(30) Priority: 05.02.1999 US 118810 P; 28.04.1999 US 131398 P; 18.05.1999 US 134674 P
(62) Divisional of application: 00907506.0
(71) Applicant: Therapeutic Human Polyclonals, Inc., Mountain View, CA 94043 (US)
(72) Inventor: Bülow, Jens-Ulrich, 76140 Karlsruhe (DE)
(74) Representative: HOFFMANN EITLE

(57) **Abstract**

Polyclonal antisera compositions are provided. These compositions are of a non-human animal. They are comprised predominantly of substantially human immunoglobulin protein molecules comprised of at least a portion of a human heavy chain polypeptide.

## Description

### INTRODUCTION

### Field of the Invention

The field of this invention is substantially human polyclonal antisera for prophylactic and therapeutic treatment of humans.

### Background

The therapy of infectious diseases caused by bacteria, fungi, virus and parasites is largely based on chemotherapy. However, the emergence of drug-resistant organisms requires the continuous development of new antibiotics. At the same time the control of infections is threatened by the emergence of new pathogens. The increasing number of immunocompromised individuals due to malnutrition, AIDS, medical therapies of cancer, autoimmune diseases and organ transplantation decreases the efficacy of antibiotic therapy and increases the difficulty of controlling infections.

Therapies of patients with malignancies and cancer are also based on chemotherapy. However, many of these therapies are ineffective and the mortality of diseased patients is high. Advances in monoclonal antibody technology have provided little improvement because of the immunogenicity of the monoclonal antibodies and their lack of potency. Anti-idiotypic antibody responses in patients undergoing monoclonal antibody therapy can render the antibody therapy ineffective.

Therapy of steroid resistant rejection of transplanted organs requires the uses of biological reagents (monoclonal or polyclonal antibody preparations) that reverse the ongoing alloimmune response in the transplant recipient. However, immunogenicity of antibody preparations may render such therapy ineffective and prevent rejection reversal. As a consequence, a transplanted organ may be rejected.
Similarly, antibody therapies of autoimmune disease patients are of limited success due to the immunogenicity of antibody preparations. While humanization of antibodies decreases immunogenicity, the effectiveness of such antibodies is limited by anti-idiotypic antibody responses and the lack of potency of monoclonal antibodies. Non-immunogenic, potent reagents for the modulation of immune responses have to be developed.

Polyclonal antibody therapy for the treatment of infectious diseases was introduced at the end of the last century. By the 1930s, serum therapy was used for treatment of bacterial and viral infections including pneumonia, meningitis, scarlet fever, whooping cough, anthrax, botulism, gangrene, tetanus, brucellosis, dysentery, tularemia, diphtheria, measles, poliomyelitis mumps and chickenpox. However, the systemic administration of animal sera caused fevers, chills, and allergic reactions. Serum sickness occurred in 10-50% of treated individuals.

The potential of using antibodies in the treatment of a variety of indications is very high. The ability to specifically bind to a target entity provides diverse opportunities to sequester and destroy the entity. However, as demonstrated above, there have been many impediments to the use of heterologous and humanized antibodies. The limitations of monoclonal antibodies adds the additional impediment of reduced affinity. Thus, there is a pressing need to find alternative modalities which provide protection against infectious disease and malignancies or the immunomodulation of transplant recipients and autoimmune disease patients.

### Relevant Literature

Antibody-based therapies in infectious diseases were recently reviewed by A. Casadevall and M. D. Scharff, Clinical Infectious Diseases 1995; 150-161.

The use of antibodies for the treatment of cancer and malignancies was recently reviewed by C. Botti, A. Marinetti, S. Nerini-Molteni, and L Ferrari, Int J Biol Markers 1997; 12(4):141-147; D.R. Anderson, A. Grillo-Lopez, C. Varns, and K.S. Chambers, Biochem Soc Trans 1997; 25(2):705-708; C.
Renner, L. Trumper, and M. Pfreundschuh, Leukemia 1997; 11 Suppl 2:S55-59; B. Bodey, S.E. Siegel, and H.E. Kaiser, Anticancer Res 1996; 16(2):661-674.

The use of polyclonal antibody preparations for the treatment of transplant rejection was recently reviewed by N. Bonnefoy-Berard and J.P. Revillard, J Heart Lung Transplant 1996; 15(5):435-442; C. Colby, C.A. Stoukides, and T.R. Spitzer, Ann Pharmacother 1996; 30(10):1164-1174; M.J. Dugan, T.E. DeFor, M. Steinbuch, and A.H. Filipovich, Ann Hematol 1997; 75(1-2):41-46.

The use of polyclonal antibody therapies for autoimmune diseases has been described by W. Cendrowski, Boll Ist Sieroter Milan 1997; 58(4):339-343; L.K. Kastrukoff, D.R. McLean, and T.A. McPherson, Can J Neurol Sci 1978; 5(2):175-178; J.E. Walker, M.M Hoehn, and N. Kashiwagi, J Neurol Sci 1976; 29(2-4):303-309.

The depletion of fat cells using antibody preparations has been described by L. De Clercq, J. Mourot, C. Genart, V. Davidts, and C. Boone, J Anim Sci 1997; 75(7):1791-1797; J.T. Wright and G.J. Hausman, Obes Res 1995; 3(3):265-272.

The cloning of animals from cells has been described by T. Wakayama, A.C.F. Perry, M. Zuccotti, K.R. Johnson and R. Yanagachi, Nature 1998; 394:369-374; J.B. Cibelli, S.L. Stice, P.J. Golueke, J.J. Kane, J. Jerry, C. Blackwell, A. Ponce de Leon, and J.M. Robl, Science 1998; 280:1256-1258; J.B. Cibelli, S. L. Stice, P. J. Golueke, J.K. Kane, J. Jerry, C. Blackwell, F. Abel de Leon, and J. Robl, Nature Biotechnology 1998; 16:642-646; A. E. Schnieke, A.J. Kind, W.A. Ritchie, K. Mycock, A.R. Scott, M. Ritchie, I. Wilmut, A. Colman A, and K.H. Campbell, Science 1997; 278(5346):2130-2133; K.H. Campbell, J. McWhir, W.A. Ritchie, and I. Wilmut, Nature 1996; 380(6569):64-66.

Production of antibodies from transgenic animals is described in U.S. Patent nos. 5,814,318; 5,545,807; and 5,570,429. Homologous recombination for chimeric mammalian hosts is exemplified in U.S. Patent no. 5,416,260. A method for introducing DNA into an embryo is described in U.S. Patent no. 5,567,607. Maintenance and expansion of embryonic stem cells is described in U.S. Patent no. 5,453,357.

### SUMMARY OF THE INVENTION

Methods are provided for the production of substantially human polyclonal antisera to a specific antigen, where a transgenic domestic animal comprising genetically altered light and heavy chain immunoglobulin loci and at least a portion of human light and heavy chain immunoglobulin loci are provided. The method employs stepwise modification of a domestic animal in which the antibody repertoire is diversified predominantly by gene conversion (e.g rabbits, sheep, pigs, cows). The method involves replacement by homologous recombination of endogenous elements of the immunoglobulin loci with the corresponding human counterparts, in particular, replacement of one or several exons encoding constant regions of heavy and light chain and one or several variable region elements including the one proximal to the D region locus. In animals, where antibody diversity is generated predominantly by gene conversion, replacement of the V region most proximal to the D region with a human V region element results in expression of the human V element in the majority of immunoglobulins. This genetic engineering is followed by breeding hosts of the same species and selecting for a host which is capable of responding to immunization with production of substantially human antisera with host glycosylation, the immunoglobulin having at least a functional portion of the human heavy chain. Animals expressing the substantially human protein sequence immunoglobulins are used for the generation of polyclonal antibody preparations by immunization with immunogens of interest, particularly, immunogens which initiate antibody production which has therapeutic activity. After purification of the antisera, such antisera may be used, by itself or in combination, with other reagents for the depletion of infectious reagents, malignant cells, cancers, undesirable target cells or immunomodulation.

### DESCRIPTION OF THE SPECIFIC EMBODIMENTS

Methods are provided for producing substantially human antisera in a heterologous host by immunizing the host with an immunogen. The host is characterized by; being at least substantially incapable of producing endogenous antisera and capable of predominantly producing substantially human polypeptide antisera upon exposure to an immunogenic substance; and retaining its capability of rearranging the immunoglobulin locus and recombining the V, (D_{H}), J and C regions to produce substantially human protein antisera, which include at least one human immunoglobulin constant region and/or at least one human variable (V) region element. Of particular interest are constant regions of the subclasses of C_{α} or C_{γ}, including any of the C_{γ} subclasses 1, 2, 3 and 4.

DNA fragments encoding human constant regions and variable elements are integrated into the genome by homologous recombination and replace the corresponding endogenous elements.

Various animals, particularly domestic animals, which can provide reasonable volumes of antisera may be employed. The animals generally are at least 1 kg, preferably 2 kg, and may be 5 kg or more when adult, although smaller animals can be used as appropriate. Also the gestation period should be less than 12 months, usually being in the range of 1 to 4 months. Illustrative animals include *Lagomorpha,* e.g. rabbit, ovine, bovine, canine, feline, equine, and the like, excluding murine. Of particular interest are animals where diversification of the antibody repertoire is accomplished predominantly by gene conversion (i.e. rabbits, pigs, sheep, cattle). In these animals, replacement of the V region element proximal to the D region with a human V region element will result in the expression of the human V region element in the majority of immunoglobulins. The described genetic engineering approach of the subject invention is substantially easier than other approaches that have been performed with mice. In mice, however, diversification of the antibody repertoire is accomplished predominantly by gene rearrangement.

Host cells, e.g. fibroblasts, keratinocytes, myocytes, hepatocytes, epithelial cells, or other cells which may be grown and expanded in culture and do not have a rearranged genome, are transformed (genetically modified) by the introduction of DNA fragments into the cells, where the fragments become integrated into the host genome. Introduction may be by a variety of methods, including bare DNA, transfection with a viral vector, fusion, biolistics, liposomes, etc. The particular method will be selected in accordance with the purpose of the introduction of the DNA and the efficiency of integration. Functional immunoglobulin light and heavy chain loci are modified by homologous recombination, by replacing at least a portion of the host heavy chain constant region with at least a functional portion of the human heavy chain constant region and if desired, analogously, the host light chain constant region with a human light chain constant region. Of particular interest is also the replacement of the V region most proximal to the D region with a human V region element. In this way, while some portions of the immunoglobulin are host sequences, the antisera is not likely to cause a strong immune response in view of the great variety of variable regions in the antisera. In animals, where antibody diversity is generated predominantly by gene conversion, replacement of the V region most proximal to the D region with a human V region element results in expression of the human V element in the majority of immunoglobulins. For the replacement of constant regions it is of particular interest to include at least about 2 of the 3 domains C_{H1}, C_{H2}, and C_{H3}, of the constant region, particularly including C_{H3}. To the extent such antisera can function in a host, particularly an immunocompromised host, the reduced number of stages to attain hosts which produce such antisera is attractive.

For integration at a predetermined site, constructs are prepared which include, in sequence, the DNA fragment for integration and a first marker gene bordered by homologous sequences of at least about 30nt and a second marker gene, whereby homologous integration results in loss of the second marker gene. By having the second marker gene providing negative selection--cells with the second marker gene are selected against and removed from the cell mixture; by having the first marker gene providing positive selection--cells having the first marker gene are retained--by using a medium to which the second marker gene is sensitive. In this manner, those cells in which the construct is randomly integrated are decreased. By following with a medium selective for the first marker gene, cells not retaining the first marker gene will be decreased. In this way, the remaining cells should be those having homologous recombination. Desirably, the cells are in a rapidly proliferating status, rather than a non-proliferating status. By employing a growth medium, such as RPMI1640 or DMEM, supplemented with FCS and growth factors, a growth cycle can be induced.

After the cells have been transformed or transfected, the cells are put in a selective medium in accordance with the marker employed, usually an antibiotic resistance or the tk gene. The cells are expanded in culture and then cloned. Individual cells in clones may then be screened for the desired genetic modification. Conveniently, PCR may be used to identify that the desired modification, deletion or integration, has taken place.

The genetic modifications may be a single modification or, if desired, after expansion of cells having the first modification, the cells may then be subjected to a second modification. For example, after replacing the heavy chain constant regions, one could replace the light chain constant regions.

Where an individual modification occurs, one can use a single marker for positive selection and use the same marker repetitively. Where two or more modifications to the same cell are generated, different positive selection markers should be used, in order to independently select at each stage. As already indicated, there are numerous antibiotic resistance genes, which genes may be used in combination, allowing for selection at each stage. Genes useful for selection include neo, tet, cam, tk, pen, mtx, etc. After the host cells have been modified and demonstrated to have the desired modification, the cells may then be fused with enucleated nuclear transfer unit cells, e.g. oocytes or embryonic stem cells, cells which are totipotent and capable of forming a functional neonate. Fusion is performed in accordance with conventional techniques which are well established. See, for example, Cibelli et al., Science (1998) 280:1256. Alternatively, enucleation of oocytes and nuclear transfer can be performed by microsurgery using injection pipettes. (See, for example, Wakayama et al., Nature (1998) 394:369) The resulting functional egg cells are then cultivated in an appropriate medium and transferred into synchronized recipients.
Another method for producing nuclear transfer unit cells is to introduce DNA constructs comprising human transgenes into fertilized eggs. The eggs may then be expanded to provide embyronic stem cells, which are screened for the desired genetic modification and subsequent embryo transfer into foster mothers, where the eggs are brought to term, and the resulting neonates screened for the modified genotype.

The resulting mutated hosts may then be used for breeding with other mutated hosts. For example, hosts having an altered,heavy chain immunoglobulin locus may be bred with hosts having an altered light chain immunoglobulin locus to breed a host capable of producing substantially human polypeptide immunoglobulins. The hemizygous siblings containing the two mutated genes are then bred to produce homozygous siblings. Homozygosity may be readily determined by the absence of the undesired gene sequences. After each breeding, the host is assayed for the presence of the genetic modification in its cells, particularly the germ cells, and may be bred to a further generation, usually not more than three generations, to ensure that the modification is stably maintained through successive generations. The genomes of the various offspring may be analyzed for the maintenance of the genetic modifications or, as appropriate, the offspring may be analyzed for the biological change which the genetic modification generated.

Once the host has been generated, the host may now be used to produce antisera under a variety of conditions. Depending upon the use of the antisera, antigens, immunogens comprising a hapten covalently bonded to an antigen, organisms, e.g. viruses and unicellular organisms, alive, attenuated or dead, fragments of organisms, organelles, cells, particularly human cells or fragments of cells, or the like may be used. Thus the antisera may be directed to an antigen, a small organic molecule or a cell, where the various entities may be endogenous or exogenous to the human host. The immunization composition may be administered in any convenient manner, with or without an adjuvant, and may be administered in accordance with a predetermined schedule. The affinity for the immunization composition may then be monitored and the antisera collected when the antisera has the desired specificity and affinity. The affinity of the antisera generally will be at least about 10⁻⁷, usually at least about 10⁻⁸, preferably at least about 10⁻⁹, or higher.

For some application, one may use hosts in which the V element proximal to the D regions has been replaced with various human V region elements. In this way, different immune responses to the same immunogen will be obtained from the different hosts, where the variable region sequence may be as a result of gene conversion, providing different alleles. The antisera from the different hosts may be mixed to provide a broader repertoiree of antibodies. Up to 10 or more different hosts may be employed, depending on the antigen of interest.

Antibody preparations are obtained by fractionating blood of genetically engineered animals expressing human sequence immunoglobulins. A concentrated immunoglobulin fraction may be prepared by chromatography (affinity, ionic exchange, gel filtration, etc.), selective precipitation with salts such as ammonium sulfate, organic solvents such as ethanol, or polymers such as polyethyleneglycol.

The fractionated antibodies may be dissolved or diluted in non-toxic, non-pyrogenic media suitable for intravenous administration in humans, for instance, sterile buffered saline. In some applications, antibody preparations may be applied directly onto epithelium. For such applications, fractionated antibodies may be dissolved in a water soluble gel such as KY-jelly and the like.

The antibody preparations used for administration are generally characterized by containing a polyclonal antibody population, having immunoglobulin concentrations from 0.1 to 100 mg/ml, more usually from 1 to 10 mg/ml. The antibody preparation may contain immunoglobulins of various isotypes. Alternatively, the antibody preparation may contain antibodies of only one isotype, or a number of selected isotypes.

In most instances the antibody preparation will consist of unmodified immunoglobulins. Alternatively, the immunoglobulin fraction may be subject to treatment such as enzymatic digestion (e.g. with pepsin, papain, plasmin, glycosidases, nucleases, etc.), heating, etc, and/or further fractionated.

The antibody preparations generally are administered into the vascular system, conveniently intravenously by injection or infusion via a catheter implanted into an appropriate vein. The antibody preparation is administered at an appropriate rate, generally ranging from about 10 minutes to about 24 hours, more commonly from about 30 minutes to about 6 hours, in accordance with the rate at which the liquid can be accepted by the patient. Administration of the effective dosage may occur in a single infusion or in a series of infusions. Repeated infusions may be administered once a day, once a week once a month, or once every three months, depending on the half-life of the antibody preparation and the clinical indication. For applications on epithelial surfaces the antibody preparations are applied to the surface in need of treatment in an amount sufficient to provide the intended end result, and can be repeated as needed.

The antibody preparations find use in their ability to bind and neutralize antigenic entities in human body tissues that cause disease or that elicit undesired or abnormal immune responses. An "antigenic entity" is herein defined to encompass any soluble or cell-surface bound molecules including proteins, as well as cells or infectious disease-causing organisms or agents that are at least capable of binding to an antibody and preferably also are capable of stimulating an immune response.

Administration of an antibody preparation against an infectious agent as monotherapy or in combination with chemotherapy results in elimination of infectious particles. A single administration of antibodies decreases the number of infectious particles generally 10 to 100 fold, more commonly more than 1000-fold. Similarly, antibody therapy in patients with malignant disease as monotherapy or in combination with chemotherapy reduces the number of malignant cells generally 10 to 100 fold, or more than 1000-fold. Therapy may be repeated over an extended amount of time to assure the complete elimination of infectious particles, malignant cells, etc. In some instances, therapy with antibody preparations will be continued for extended amounts of time in the absence of detectable amounts of infectious particles or undesirable cells. Similarly, the use of antibody therapy for the modulation of immune responses may consist of single or multiple administrations of therapeutic antibodies. Therapy may be continued for extended amounts of time in the absence of any disease symptoms.

The subject treatment may be employed in conjunction with chemotherapy at dosages sufficient to inhibit infectious disease or malignancies. In autoimmune disease patients or transplant recipients antibody therapy may be employed in conjunction with immunosuppressive therapy at dosages sufficient to inhibit immune reactions.

The following examples are offered by way of illustration and not by way of limitation.

### EXPERIMENTAL

### Generation of transgenic rabbits expressing substantially human immunoglobulin

Exons encoding human constant region elements and variable region elements are integrated into the genome of rabbit fibroblasts by homologous recombination. Rabbit fibroblasts are transfected with various linearized DNA constructs containing human immunoglobulin locus elements. Successfully transfected cells are selected and used for the cloning of rabbits.

### Cloning of rabbits

Mature Dutch Belton rabbits are superovulated by subcutaneous injection of follicle stimulating hormone (FSH) every 12 hours (0.3 mg x 2 and 0.4 mg x 4). Ovulation is induced by intravenous administration of 0.5 mg luteinizing hormone (LH) 12 hours after the last FSH injection. Oocytes are recovered by ovidual flush 17 hours after LH injection. Oocytes are mechanically enucleated 16-19 hours after maturation. Chromosome removal is assessed with bisBENZIMIDE (HOECHST 33342, Sigma, St. Louis, MO) dye under ultraviolet light.

Enucleated oocytes are fused with actively dividing fibroblasts by using one electrical pulse of 180 V/cm for 15 us (Electrocell Manipulator 200, Genetronics, San Diego, CA). After 3-5 hours oocytes are chemically activated with calcium ionophore (6 uM) for 4 min (# 407952, Calbiochem, San Diego, CA) and 2 mM 6-dimethylaminopurine (DMAP, Sigma) in CR2 medium (Specialty Media, Lavalett, NJ) with 3 mg/ml bovine serum albumin (fatty acid free, Sigma) for 3 hours. Following the activation, the embryos are washed in hamster embryo culture medium (HECM)-Hepes five times and subsequently, cultivated in CR2 medium containing 3 mg/mgl fatty-acid free BSA for 7 days at 37.8° C and 5%CO2 in air. Embryos are then transferred into synchronized recipients. Offsprings are analyzed by PCR for a segment of the transgene.

### Binding of human antibodies expressed in rabbits to Hepatitis B surface antigen

Genetically engineered rabbits (as described above) are immunized intramuscularly with purified Hepatitis B surface antigen (HBsAg) (10µg in incomplete Freund's adjuvant) on day 0 and day 14. On day 28 animals are bled from the ear and serum is prepared. ELISA plates (NUNC, Denmark) are coated with 1 µg/ml HBsAg in PBS for 1 hour at room temperature. Subsequently, available binding sites are blocked by incubation with 1% non-fat dry milk (NFM) in PBS (300 µl/well). Rabbit serum is diluted in PBS/1%NFM and added to the coated wells. After an incubation of 1 hour, the plates are washed 3 times with PBS/0.05% Tween 20 and bound Ig is detected using goat anti-human Ig conjugated with horse-radish peroxidase. Conjugated goat antibody is detected using o-phenylenediamine dihydrochloride (Sigma) at 1 mg/ml. The colorimetric reaction is stopped by addition of 1 M HCl solution and the absorbance is measured at 490 nm. As a control serum from non-immunized rabbits is used. Serum from non-immunized rabbits does not react with HBsAg. At a dilution of 1:100 the optical density measured in uncoated and HBsAg coated wells is below 0.4. In contrast, serum from immunized rabbits contains substantially human antibodies reactive with HBsAg. At a serum dilution of 1:100 the measured optical density is 2.8. Upon further dilution of the serum the measured optical density declines to 0.2 (at a dilution of 25600). No antibodies reactive with a goat anti-rabbit IgG-HRP conjugate can be detected. This demonstrates that the genetically engineered rabbits produce substantially human anti-HBsAg antibodies following immunization.

### Complement mediated cytotoxicity of virus infection cell line using human antibodies

A human liver carcinoma cell line expressing HBsAg is labeled with 0.1 mCi ⁵¹Cr in 100 ul PBS for 1 hr at 37°C. Two thousand ⁵¹Cr-lableled cells are incubated with serum from genetically engineered rabbits expressing anti-HBsAg immunoglobulin (see above). After two hours at 37°C the release of ⁵¹Cr into the supernatant is determined by measuring radioactivity using a scintillation counter. For the determination of maximum release, 1% Triton X100 is added. The degree of cell lysis is calculated as follows: %Lysis = CPM experimental ±CPM#spontaneous / CPM# total ± CPM spontaneous. Incubation of labeled cells with serum (diluted 1:30) from non-immunized rabbits does not result in cell lysis (<10%). However, incubation of cells with serum from immunized rabbits causes 80% cell lysis. Inactivation of complement in the serum by heat treatment (56°C for 30 minutes) renders the serum from immunized rabbits inactive. These results demonstrate that substantially human antibodies produced by genetically engineered rabbits bind to HBsAg-positive cells and cause complement dependent lysis.

### Treatment of animal with infection.

Substantially human immunoglobulin is purified from the serum of genetically engineered rabbits by ammonium sulfate precipitation and ion exchange chromatography. SCID-mice are injected with one million human liver carcinoma cells expressing HBsAg. Subsequently, 25 µg immunoglobulin is injected peritoneally once per day. Animals treated with antibodies isolated from non-immunized rabbit serum die after about 60 days. This is similar to untreated recipients of liver carcinoma cells. In contrast, mice treated with antibodies isolated'from immunized rabbit serum survive for more than 150 days. This demonstrates that human antibodies produced in genetically engineered rabbits are capable of eliminating human carcinoma cells from SCID-mice.

It is evident from the above results that by using genetically engineered rabbits expressing substantially human immunoglobulin genes, polyclonal antibody preparations against antigens, infectious particles, cancer cells, and the like can be generated. Such polyclonal antibody preparations can be used to treat patients suffering from an infectious disease or a malignancy. The antisera also can be used to modulate an immune response by elimination of cell subpopulations, cytokines, or the like. The human antibody preparation has a substantially reduced likelihood of engendering an immune response in human patients, as compared to heterologous antisera, it will have few side effects and it can be used safely with positive results.

All of the references cited herein are incorporated herein by reference as if each reference was individually wholly incorporated.

It will be apparent to one of ordinary skill in the art that many changes and modifications can be made thereto without departing from the spirit or scope of the appended claims.

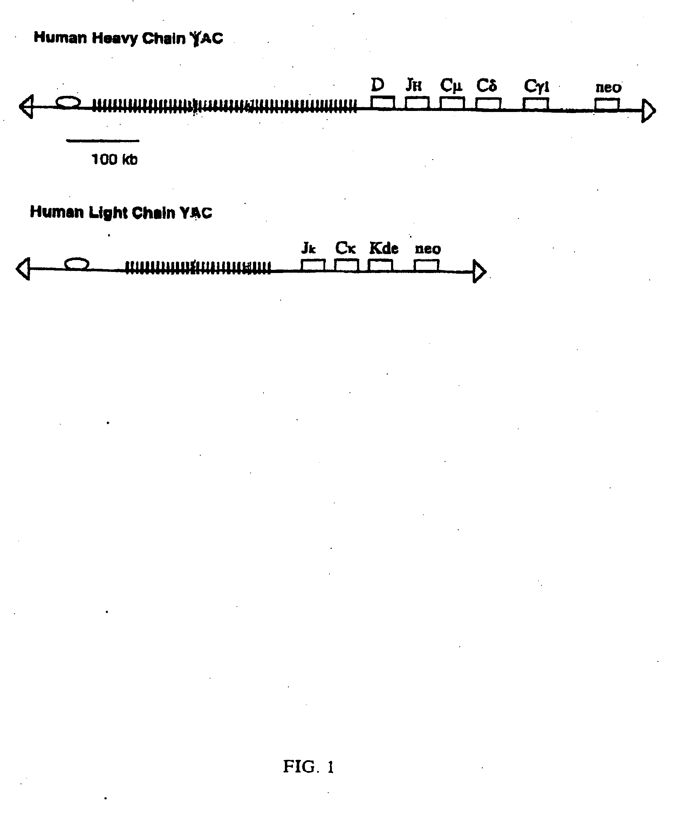

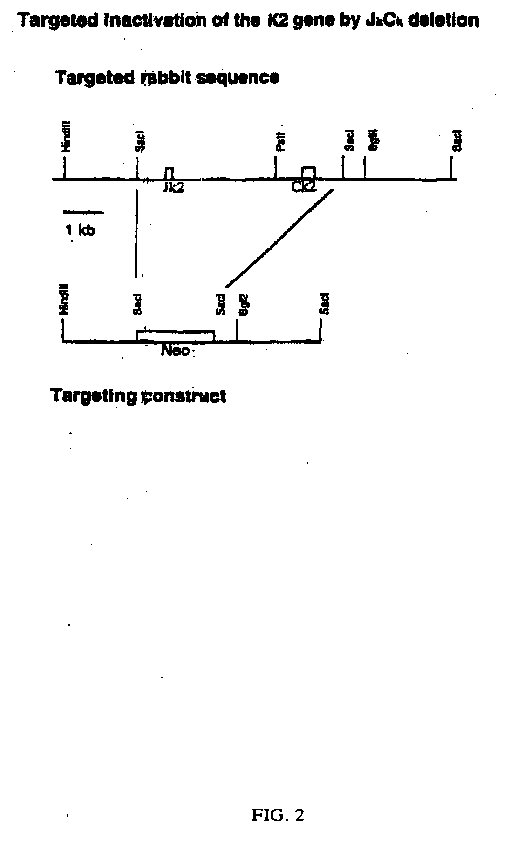

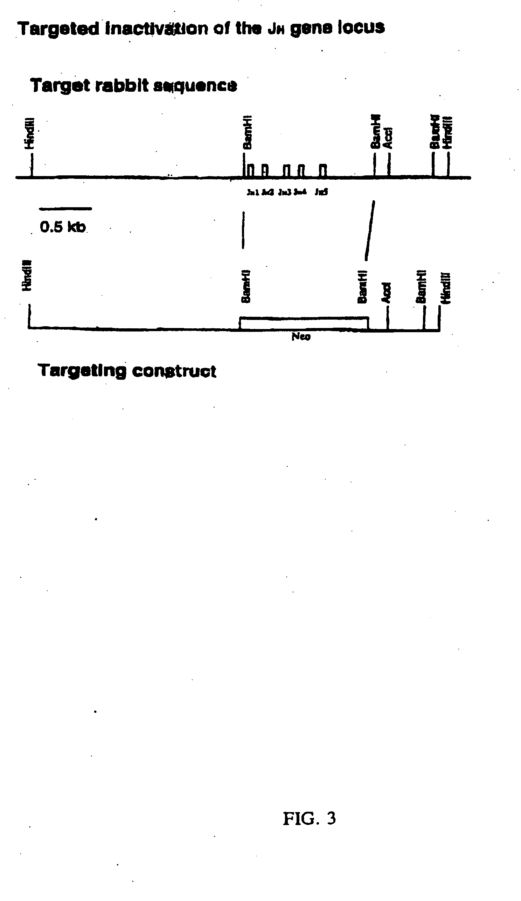

## Claims

1. A polyclonal antisera composition of a nonhuman animal that specifically recognizes an immunogen, wherein said antisera composition is comprised predominantly of substantially human immunoglobulin protein molecules comprised of at least a portion of a human heavy chain polypeptide, wherein said substantially human immunoglobulin protein molecules specifically bind to said immunogen.

2. The polyclonal antisera according to Claim 1, wherein said transgenic nonhuman animal is immunized with said antigenic entity, weighs at least 1 kg and comprises at least a portion of functional human heavy chain immunoglobulin genes integrated by homologous recombination into its genome.

3. The polyclonal antisera composition according to Claim 1, wherein said transgenic nonhuman animal generates antibody diversity predominately by gene conversion.

4. The polyclonal antisera composition according to Claim 1, wherein said transgenic nonhuman animal is from the order *Lagomorpha.*

5. The polyclonal antisera composition according to Claim 1, wherein said portion of functional human heavy chain immunoglobulin genes comprises at least one constant region element.

6. The polyclonal antisera composition according to Claim 5, wherein said portion of functional human heavy chain immunoglobulin genes further comprises at least one variable region element.

7. The polyclonal antisera composition according to Claim 6, wherein said variable region element is the variable region element proximal to the D region.

8. The polyclonal antisera composition according to Claim 1, wherein said immunogen comprises a disease causing organism or antigenic portion thereof.

9. The polyclonal antisera composition according to Claim 1, wherein said immunogen is an antigen endogenous to humans.

10. The polyclonal antisera composition according to Claim 1, wherein said immunogen is an antigen exogenous to humans.

11. A transgenic nonhuman animal weighing at least 1 kg and comprising at least a portion of functional human heavy chain immunoglobulin genes integrated by homologous recombination into its genome, wherein said portion of functional human heavy chain immunoglobulin genes rearranges in frame with heavy chain immunoglobulin sequences endogenous to said nonhuman animal to encode functional, substantially human antibody molecules that comprise at least in part human heavy chain immunoglobulin polypeptide sequences, and wherein said animal predominantly produces said functional, substantially human antibody molecules when immunized.

12. A transgenic nonhuman animal weighing at least 1 kg and comprising at least a portion of functional human light chain immunoglobulin genes integrated by homologous recombination into its genome, wherein said human light chain immunoglobulin genes rearrange in frame with sequences endogenous to said nonhuman animal to encode functional, substantially human antibody molecules that comprise at least in part human light chain immunoglobulin polypeptide sequences.

13. The transgenic nonhuman animal according to Claim 11 or 12, wherein said transgenic nonhuman animal generates antibody diversity predominately by gene conversion.

14. The transgenic nonhuman animal according to Claim 11 or 12, wherein said transgenic nonhuman animal is from the order *Lagomorpha*.

15. The transgenic nonhuman animal according to Claim 11 or 12, wherein said portion of functional human heavy chain immunoglobulin genes comprises at least one constant region element.

16. The transgenic nonhuman animal according to Claim 15, wherein said portion of functional human heavy chain immunoglobulin genes further comprises at least one variable region element.

17. The transgenic nonhuman animal according to Claim 16, wherein said variable region element is the variable region element proximal to the D region.

18. The transgenic nonhuman animal according to Claim 12, wherein said human immunoglobulin light chain gene encodes the κ chain.

19. An antisera composition produced by the transgenic nonhuman animal according to Claim 11.

20. A method for neutralizing an antigenic entity in a human body component, said method comprising:
contacting said body component with an antisera composition according to Claim 1, whereby said substantially human immunoglobulin protein molecules in said antisera composition specifically bind and
neutralize said antigenic entity.

21. The method according to Claim 20, wherein said antigenic entity is from an organism that causes an infectious disease.

22. The method according to Claim 20, wherein said antigenic entity is a cell surface molecule.

23. The method according to Claim 22, wherein said cell surface molecule is from a lymphocyte or an adipocyte.

24. The method according to Claim 20, wherein said antigenic entity is a human cytokine or a human chemokine.

25. The method according to Claim 20, wherein said antigenic entity is a cell surface molecule an a malignant cancer cell.

26. A method of producing a transgenic nonhuman animal weighing at least 1 kg and comprising human immunoglobulin genes integrated by homologous recombination into its genome, wherein said animal predominantly produces functional, substantially human antibody molecules comprised at least in part of human immunoglobulin polypeptide sequences when immunized, said method comprising:
producing a first mutated animal comprising heavy chain immunoglobulin loci where constant and/or variable region elements are replaced with at least a functional portion of the human heavy chain immunoglobulin locus by genetic alteration of a cell nucleus of said animal, introducing said cell nucleus into an enucleated nuclear transfer unit cell to provide a first embryonic stem cell, introducing said first nuclear transfer unit cell into a female recipient host to produce a first mutated neonate;
producing a second mutated animal comprising light chain immunoglobulin loci where constant and/or variable region elements are replaced with at least a functional portion of the human light chain immunoglobulin locus by genetic alteration of a cell nucleus of said animal, introducing said cell nucleus into an enucleated nuclear transfer unit cell to provide a second embryonic cell stem cell, introducing said second nuclear transfer unit cell into a female recipient host to produce a second mutated neonate; and
breeding mature first and second mutated neonates and selecting animals capable of producing substantially human antisera and being at least substantially incapable of producing endogenous antisera.

27. A method of producing a transgenic nonhuman animal weighing at least 1 kg and comprising human immunoglobulin genes integrated by homologous recombination into its genome, wherein said animal predominantly produces functional, substantially human antibody molecules comprised at least in part of human immunoglobulin polypeptide sequences when immunized, said method comprising:
producing a mutated animal comprising heavy and light chain immunoglobulin loci where constant and/or variable region elements are replaced with at least a functional portion or the human heavy and/or light chain immunoglobulin locus by genetic alteration of a cell nucleus of said animal, introducing said cell nucleus into an enucleated nuclear transfer unit cell to provide a embryonic cell stem cell, introducing said nuclear transfer unit cell into a female recipient host to produce mutated neonate; and
breeding mature mutated neonates and selecting animals capable of producing substantially human antisera and at least substantially incapable of producing endogenous antisera.

28. The method according to Claim 26 or 27 wherein said nuclear transfer unit cell is an oocyte.

29. The method according to Claim 26 or 27, wherein said animal is from the order of *Lagomorpha.*

30. A method according to Claim 26 or 27, wherein said heavy chain locus comprises at least one constant region element.

31. A method according to Claim 26 or 27, wherein said heavy chain locus comprises at least one variable region element.

32. A method according to Claim 26 or 27, wherein said heavy chain locus comprises the variable region element proximal to the D region.
